(19) Europäisches Patentamt
European Patent Office
Office européen des brevets

(11) **EP 4 618 046 A1**

(12) **EUROPEAN PATENT APPLICATION**
published in accordance with Art. 153(4) EPC

(43) Date of publication:
**17.09.2025   Bulletin 2025/38**

(21) Application number: 23892053.2

(22) Date of filing: **16.11.2023**

(51) International Patent Classification (IPC):
*G06V 20/69* (2022.01)        *G06V 10/80* (2022.01)
*G06V 10/774* (2022.01)       *G06V 30/19* (2022.01)
*G06V 10/46* (2022.01)        *G06V 10/82* (2022.01)

(52) Cooperative Patent Classification (CPC):
**G06N 3/08; G06V 10/46; G06V 10/764;
G06V 10/774; G06V 10/80; G06V 10/82;
G06V 20/69; G06V 30/19; G06V 30/42**

(86) International application number:
**PCT/KR2023/018499**

(87) International publication number:
**WO 2024/106990 (23.05.2024 Gazette 2024/21)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **18.11.2022   KR 20220155020**

(71) Applicant: **LG Management Development Institute
Co., Ltd.
Seoul 07336 (KR)**

(72) Inventor: **JANG, Jong Seong
Seoul 07796 (KR)**

(74) Representative: **BCKIP Part mbB
MK1
Landsbergerstraße 98, 3.Stock
80339 München (DE)**

(54) **METHOD AND SYSTEM FOR TRAINING IMAGE CLASSIFICATION MODEL FOR MULTI-LABEL IMAGES, AND METHOD FOR CLASSIFYING IMAGES THROUGH IMAGE CLASSIFICATION MODEL**

(57)    A method whereby a computing system including a memory and a processor trains an image classification model, according to an embodiment of the present invention, comprises the steps of: augmenting text data in sample data of a plurality of image-text pairs; training the image classification model by fine-tuning a pretrained vision-language model on the basis of the data-augmented texts and images; and providing the trained image classification model.

**FIG. 4**

AUGMENT IMAGE-TEXT DATA, AND GENERATE POSITIVE PAIRS AND NEGATIVE PARIS — S101

EXTRACT FEATURE BY ENCODING IMAGE-TEXT — S103

GENERATE SIMILARITY METRIC OF IMAGE-TEXT FEATURE ACCORDING TO POSITIVE/NEGATIVE PAIRS — S105

PERFORM TRAINING BY APPLYING RELAXED LOSS BETWEEN POSITIVE PAIRS TO GENERATED SIMILARITY METRIC — S107

PERFORM MULTI-LABEL CLASSIFICATION TASK OF INPUT IMAGE BASED O N IMAGE CLASSIFICATION MODEL INCLUDING TRAINED NEURAL NETWORK — S109

PROVIDE MULTI-LABEL CLASSIFICATION TASK RESULT CLASSIFIED BY IMAGE CLASSIFICATION MODEL — S111

**Description**

**[TECHNICAL FIELD]**

The present invention as a machine learning model related technology including a neural network relates to a method and a system for training an image classification model for multi-label images, and a method for classifying images through the image classification model.

**[BACKGROUND ART]**

Techniques for pathology classification of medical images with deep neural networks have been successfully developed in various areas.

However, it is difficult to construct a large-scale, high-quality training data set for robustly training a deep neural network because a labeling task for a medical image is required for training an existing deep neural network, and deep medical knowledge is required for labelers.

However, with recent emergence of a pretrained deep neural network for large-scale general domain data, solutions to the problems have been proposed using a deep neural network pretrained with general domain data.

In particular, contrastive language-Image pretraining (CLIP) proposes a contrastive pretraining scheme for self-supervised learning of a general image-text pair, and dramatically improves a pretraining performance of a visual transformer.

Based on Prior Art Document 1, Prior Art Document 2 (CheXzero: Expert-level detection of pathologies from unannotated chest x-ray images via self-supervised learning) has developed a model for classifying the pathology of a chest x-ray image by fine-tuning a chest x- ray image and a pathology annotation thereto into a pair of contrastive image-texts using a contrastive image-text encoder.

Prior Art Document 2 fine-tunes the pretrained visual transformer of Prior Art Document 1 with more than 200,000 pairs of chest X-ray images and diagnostic reports for each X-ray image, and performs a zero-shot pathology analysis with the fine-tuned model, showing a diagnostic accuracy level similar to that of an authorized radiologist. That is, Prior Art Document 1 shows that pathology classification can be performed with a minimal training data set without a labeled image-text pair with a medical image, which is an existing training data set.

However, the pathology classification model of Prior Art Document 1 is fine-tuned to the same contrastive training framework as Prior Art Document 1, and has a disadvantage of not being able to analyze a multi-label nature for the medical image. In detail, the pathology classification model of Prior Art Document 2, which is pretrained with the same contrastive training objective as the zero-shot analysis scheme of Prior Art Document 1, has a problem in that a plurality of pathologies among lung opacity, edema, pneumonia, and other complications are not detected for one chest X-ray examination image.

Prior Art Document 3 (MedCLIP: Contrastive learning from unpaired medical images and text) for another pathology classification model partially deals with the problem of multi-labeling for images, but as a method for solving this problem, fine tuning is performed on a medical image through an expert's labeled training data set, and there is a problem in that fine tuning is difficult because it is difficult to sufficiently obtain expert's labeled data.

On the other hand, a research to augmenting limited training data is also active.

For data augmentation, it is common to add sample data in such a scheme that the image is subjected to various random deformations (e.g., cropping, horizontal flipping, affine transform, color jittering, and Gaussian blurring) and then matched again with an existing text. In this case, metadata may be further used. For example, Prior Art Document 5 (MedAug) discloses a technology of performing training with a similar positive pair of images including the same study ID, and Prior Art Document 6 (MICLe) discloses a technology of fine-tuning by identifying an image of the same person using metadata and giving a weight during pretraining. Prior Art Documents 5 and 6 described above disclose a method in which, in a case where an image for the same study ID and the same person is insufficient, the image is modified to augment data, and the augmented data is trained to be a positive pair.

Prior Art Document 7 (MCL: Masked Contrastive Learning) discloses a training scheme having a new loss function so that data augmentation or similarity of data determined as a positive pair on metadata is high.

[Prior Art Document]

Prior Art Document 1: Alec Radford, Jong Wook Kim, Chris Hallacy, Aditya Ramesh, Gabriel Goh, Sandhini Agarwal, Girish Sastry, Amanda Askell, Pamela Mishkin, Jack Clark, Gretchen Krueger, Ilya Sutskever. Learning Transferable Visual Models From Natural Language Supervision, on 26 Feb 2021

Prior Art Document 2: Ekin Tiu, Ellie Talius, Pujan Patel, Curtis P Langlotz, Andrew Y Ng, and Pranav Rajpurkar. Expert-level detection of pathologies from unannotated chest x-ray images via selfsupervised learning. Nature Biomedical Engineering, pages 1?8, 2022.

Prior Art Document 3: Zifeng Wang, Zhenbang Wu, Dinesh Agarwal, and Jimeng Sun. Medclip: Contrastive learning from unpaired medical images and text. In EMNLP, 2022.

Prior Art Document 4: Yuhao Zhang, Hang Jiang, Yasuhide Miura, Christopher D Manning, and Curtis P Langlotz. Contrastive learning of medical visual representations from paired images and text. arXiv preprint arXiv:2010.00747, 2020.

Prior Art Document 5: Yen Nhi Truong Vu, Richard Wang, Niranjan Balachandar, Can Liu, Andrew Y Ng, and Pranav Rajpurkar. Medaug: Contrastive learning leveraging patient metadata improves representations for chest x-ray interpretation. In Machine Learning for Healthcare Conference, pages 755?769. PMLR, 2021.

Prior Art Document 6: Shekoofeh Azizi, Basil Mustafa, Fiona Ryan, Zachary Beaver, Jan Freyberg, Jonathan Deaton, Aaron Loh, Alan Karthikesalingam, Simon Kornblith, Ting Chen, et al. Big self-supervised models advance medical image classification. In Proceedings of the IEEE/CVF International Conference on Computer Vision, pages 3478?3488, 2021.

Prior Art Document 7: Maria de la Iglesia-Vay'a. Padchest: A large chest x-ray image dataset with multi-label annotated reports. Medical image analysis, 66:101797, 2020.

## [DETAILED DESCRIPTION OF INVENTION]

### [TECHNICAL PROBLEMS]

In order to solve the above-described problems, the present invention provides an image classification model that accurately performs each label classification for a multi-label nature by fine-tuning a vision language model (e.g., CLIP) pretrained with contrastive supervising.

Further, the present invention provides an image classification method that may perform classification for each of multiple labels for images of a category having the multi-label nature by using the image classification model.

For example, the present invention provides a pathology classification method in which the image classification model performs diagnosis for a plurality of pathologies of a medical image.

In particular, a method for contrastively training multi-label images provides a new fine tuning technique that may classify pathologies by zero shots by considering a multi-label nature of a medical image text pair.

Specifically, the method for contrastively training multi-label images of the present invention provides a new fine-tuning strategy based on sentence sampling and positive-pair loss mitigation to improve the self-stream zero-shot pathology classification performance.

### [TECHNICAL SOLUTION]

According to an embodiment of the present invention, a method for training image classification model by a computing system including a memory and a processor includes: augmenting text data in sample data of a plurality of image-text pairs; training the image classification model by fine-turning a pretrained vision-language model based on the data-augmented texts and an image; and providing the trained image classification model.

According to an embodiment of the present invention, a computing device for individually classifying a plurality of pathologies for an input medical image having a multi-label nature includes: a memory storing an image classification model classifying a plurality of pathologies for the input medical image; and one or more processors operatively combining the image classification mode and the data of the memory, and

the one or more processors obtain raw data composed of a plurality of multi-label images, and reports including descriptions of the multi-label images, detect a multi-label image according to a preset criterion in the obtained raw data, detect a report associated with the detected multi-label image, individually separating sentences included in the plurality of reports to detect n sentences, augment text data by generating a plurality of active directories based on the n detected sentences, train an image classification model by fine-turning a vision-language model pretrained based on the data-augmented texts and an image, and provide the trained image classification model.

### [EFFECT OF INVENTION]

A method for training an image classification model for a multi-label image according to the present invention can provide an image classification model with improved downstream zero-shot performance by fine-tuning an image classification model pretrained with a general domain image-text pair into a training data set without a separate labeling task.

The image classification model and the image classification method using the same according to the present invention can accurately perform redundant classification by determining whether each label is applied to an image having a multi-label nature.

Specifically, when the pretrained vision-language model is fine-turned by the image classification training method according to the embodiment, it can be confirmed that the AUROC is improved by 5.77% as a result of performing zero-shot pathology classification on average. In addition, the image classification model of the present invention shows a performance much similar to or better than that of a radiologist certified by a committee.

Specifically, it can be confirmed that the image classification model of the present invention performs image label classification with relatively high accuracy in the zero-shot classification for five major diseases of a CheXpert dataset (F1 score: 0.619 to 0.625, MCC: 0.530 to 0.544).

**[BRIEF DESCRIPTION OF THE DRAWING]**

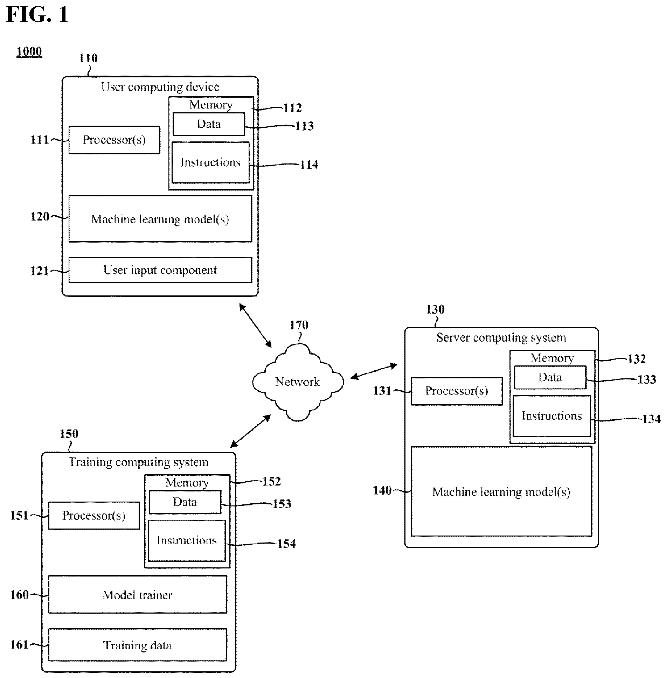

FIG. 1 illustrates an example of a block diagram of a computing system for training and controlling an image classification model that effectively and accurately performs image classification of multiple labels according to an embodiment of the present invention.

FIG. 2 illustrates an example of a block diagram of a computing device for training and controlling an image classification model that effectively and accurately performs image classification of multiple labels according to an embodiment of the present invention.

FIG. 3 illustrates an example of a block diagram in another aspect for a computing device for training and controlling an image classification model that effectively and accurately performs image classification of multiple labels according to an embodiment of the present invention.

FIG. 4 is a flowchart of a method for training an image classification model and a method for classifying an image using the trained image classification model according to an embodiment of the present invention.

FIG. 5 includes a conceptual diagram (a) illustrating a relationship between an embedding vector of a medical image and an embedding vector of a diagnosis report of the medical image, and a conceptual diagram (b) illustrating a relationship between the embedding vector of the medical image and an embedding vector of data magnified active directories from the diagnosis report of the medical image.

FIG. 6 illustrates an example illustrating a text data magnification method according to an embodiment of the present invention.

FIG. 7 illustrates an example of a state in which an image classification model is trained according to an embodiment of the present invention.

FIG. 8 illustrates an example of a state of classifying an image through the trained image classification model according to an embodiment of the present invention.

FIG. 9 illustrates an example in which the image classification model provides a result of performing classification of images of multiple labels according to an embodiment of the present invention.

**[BEST MODE FOR CARRYING OUT THE INVENTION]**

Examples of image types which may be used may include a video frame, LiDAR point cloud, an X-ray image, a computer tomography scan, a hyperspectral image, and/or various other types of images.

In an embodiment, the training data 160 is composed of an image-text pair, and the image may be an image with a multi-label nature that may correspond to at least one of the multi-labels, and the text may include information about whether the image corresponds to each of the multi-labels in a report that includes a description of the image.

As such, the text in the image-text pair may refer to a text detected in association with an image in sample data according to a specific criterion for self-supervised learning with sentences associated with the image. That is, the text may mean a text associated with an image among texts included in the sample data, which is not a label generated by a labeler in a labeling task on the image.

For example, a diagnosis report for a medical image may be an image-text pair of training data, and more specifically, may mean a bundle of a lung radiation image and a pathology diagnosis report prepared by an expert for a lung radiation image.

The training data 160 and image classification target data may be provided by a user computing device 110 and/or a server computing system 130. When the training computing device 150 trains the machine learning model 120 with respect to specific data of the user computing device 110, the machine learning model may be characterized as a personalized model.

In addition, the model trainer 160 includes a computer logic utilized to provide a desired function. The model trainer 160 may be implemented as hardware, firmware, and/or software controlling a universal processor. For example, in an embodiment, the model trainer 160 may include a program file stored in a storage device, and may be loaded to the memory 152 and executed by one or more processors 151.

The network 170 includes a 3rd Generation Partnership Project (3GPP) network, a Long Term Evolution (LTE) network, a World Interoperability for Microwave Access (WIMAX) network, Internet, a Local Area Network (LAN), Wireless Local Area Network (LAN), a Wide Area Network (WAN), a Personal Area Network (PAN), a Bluetooth network, a satellite broadcasting network, an analog broadcasting network, and/or a Digital Multimedia Broadcasting (DMB) network, etc., but is not limited thereto.

In general, communication through the network 170 may be performed through various communication protocols (e.g., TCP/IP, HTTP, SMTP, and FTP), encoding or formats (e.g., HTML and XML, etc.), and/or protective schemas (e.g., VPN, secure HTTP, and SSL) by using uses any type of wired and/or wireless connection.

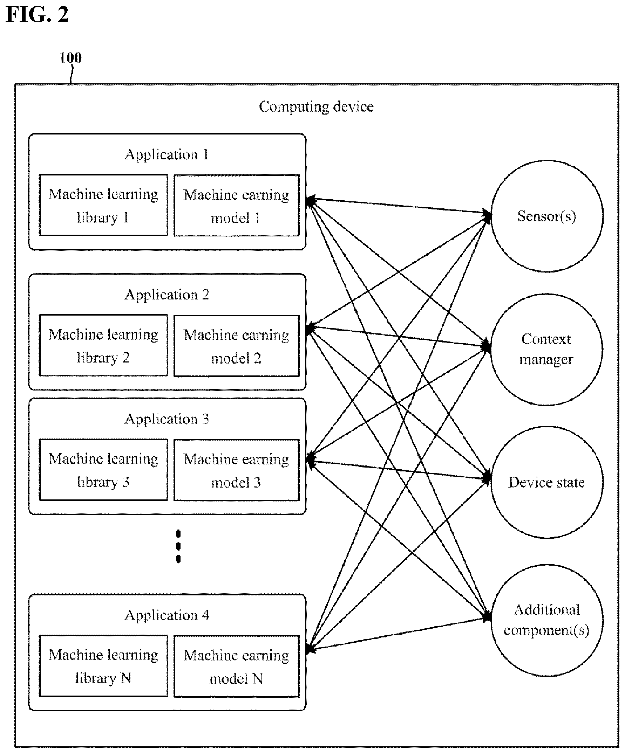

FIG. 2 illustrates an example of a block diagram of a computing device for training and controlling an image classification model that effectively and accurately performs image classification of multiple labels according to an embodiment of the present invention.

Referring to FIG. 2, the computing device 100 included in the user computing device 110, the server computing system 130, and the training computing system 150 includes multiple applications (e.g., applications 1 to N). Each application may include a machine learning library and one or more machine learning models. For example, the application may include an image processing (e.g., detection, classification, segmentation, etc.) application, a text messaging application, an email application, a dictation application, a virtual keyboard application, a browser application, a chat-bot application, etc.

In an embodiment, the computing device 100 may include a model trainer 160 for training an image classification model, and may store and operate the trained image classification model to perform image classification for an input image.

Each application of the computing device 100 may communicate with, for example, multiple other components of the computing device 100, such as one or more sensors, context managers, device state components, and/or additional components. In an embodiment, each application may communicate with each device component by using an API (e.g., a public API). In an embodiment, the API used by each application may be specific for the corresponding application.

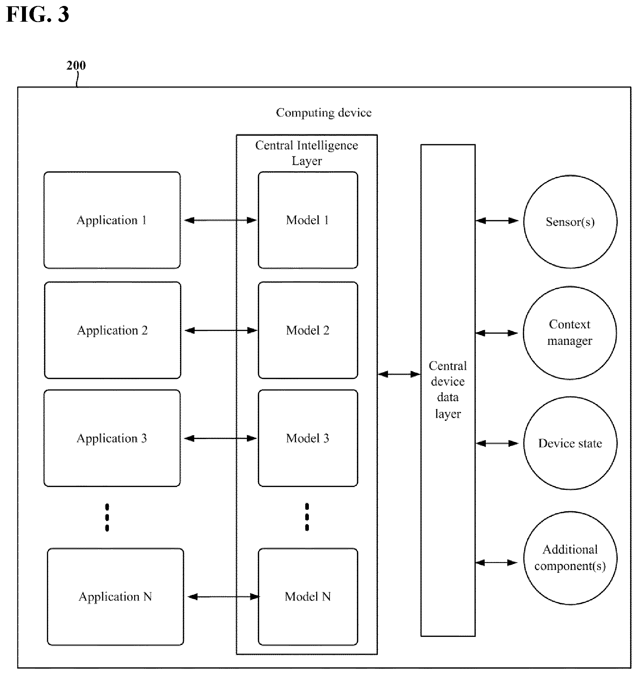

FIG. 3 illustrates an example of a block diagram in another aspect for a computing device 100 for training and controlling an image classification model that effectively and accurately performs image classification of multiple labels according to an embodiment of the present invention.

Referring to FIG. 3, the computing device 100 includes multiple applications (e.g., applications 1 to N). Each application may communicate with a central intelligence layer. For example, the applications may include an image processing application, a text message application, an email application, a dictation application, a virtual keyboard application, a browser application, etc. In an embodiment, each application may communicate with the central intelligence layer (and a model stored therein) by using an API (e.g., a common API for all applications).

The central intelligence layer may include multiple machine learning models. For example, as illustrated in FIG. 3, at least some of the respective machine learning models may be provided for each application, and managed by the central intelligence layer. In another implementation example, two or more applications may share a single machine learning model. For example, in some implementation examples, the central intelligence layer may provide the single model for all applications. In some implementation examples, the central intelligence layer may be included in an operating system of the computing device 100 or implemented differently therefrom.

The central intelligence layer may communicate with a central device data layer. The central device data layer may be a centralized data storage for the computing device 100. As illustrated in FIG. 3, the central device data layer may communicate with multiple other components of the computing device 100, such as one or more sensors, context managers, device state components, and/or additional components, for example. In some implementation examples, the central device data layer may communicate with each device component by using an API (e.g., a private API).

The techniques describe in this specification may refer to the information sent from the servers, databases, software applications and other computer-based systems, as well as the taken action and the information transmitted to the system or transmitted from the system. An intrinsic flexibility of the computer-based systems will perceive that extensive possible components, combinations, division of combinations and tasks and a functionality between components and from the components are permitted. For example, the processes described in this specification may be implemented by using a single device or component or multiple devices or components which operate as combinations. Databases and applications may be implemented in a single system or components distributed over multiple systems. The distributed components may operate in sequence or in parallel.

**[Image Classification Model Training Method and Image Classification Method Using Image Classification Model]**

Hereinafter, a method in which the computing system 1000 that trains/operates such an image classification model trains the image classification model and performs image classification through the trained image classification model will be described in detail.

Here, an image to be classified may be an image having a multi-label nature in which it is individually determined whether applications are individually applied to a plurality of labels, respectively. For example, a medical image (e.g., a chest X-ray examination image) including an X-ray image, a computed tomography scan image, and the like may be diagnosed for a plurality of pathologies (e.g., lung opacity, edema, and pneumonia), respectively, so that a plurality of pathologies that may be diagnosed for the medical image may correspond to a plurality of labels.

In the following description, the medical image is represented and described by the image having the multi-label nature, and a label for the medical image is described with reference to diagnostic pathologies for the medical image.

The computing system 1000 according to the present invention trains the image classification model by fine-tuning the pretrained vision-language model through the sample data (image-text pair) for the medical image, effectively training the image classification model capable of accurately classifying the medical image of the multi-label nature, resulting in a consistent improvement in the multiple pathology classification for the zero-shot medical image, and may perform the pathology classification with better accuracy than the certified radiologist of the committee.

Here, the pretrained vision-language model may be contrastive trained models from image-text pairs in the general domain, such as contrastive language-image pretraining (CLIP), BigGAN, self-supervision meets language-image pretraining (Slip) or/and unified framework for contrastive language-image pretraining (Uniclip).

The fine tuning technique in the image classification model training method in the embodiment of the present invention may 1) perform data augmentation to solve a problem of insufficient sample data. In this way, a labeling task (e.g., an annotation task) for the medical image is not required, and fine tuning of the image classification model may be smoothly performed by extracting only texts required for the pathology classification for the medical image, and augmenting the extracted texts to obtain multiple sample data.

In addition, the fine tuning technique in the image classification model training method in the embodiment of the present invention may further improve classification accuracy for each label for the medical image having a multi-label nature by 2) applying a relaxing loss function that relaxes a vector similarity between positive pairs of image-texts.

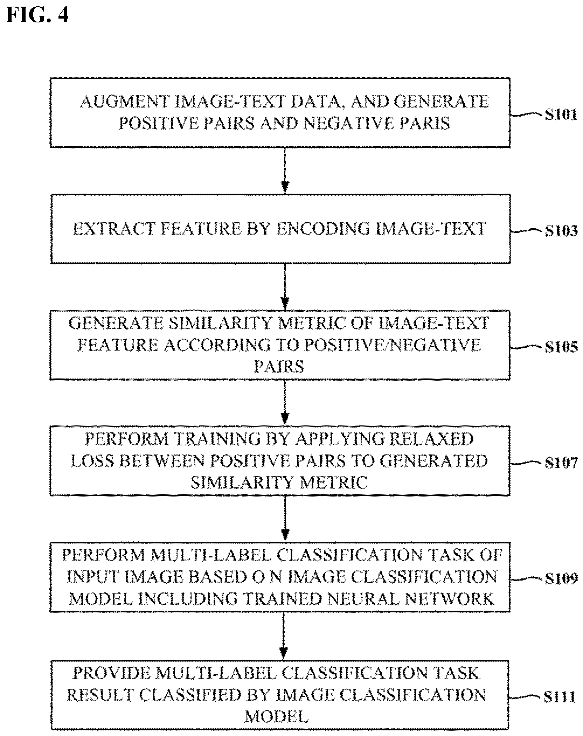

Hereinafter, the image classification model training method will be described in more detail with reference to FIGS. 4 to 7.

The image classification model training method according to the embodiment may include a step S101 of generating sample data, a step S103 of encoding each image-text of the sample data to output an embedding, a step S105 of generating a similarity metric between the output image embedding and the text embedding as a positive/negative pair, and a step S107 of training the positive pair to have a similarity based on a loss function obtained by relaxing the generated similarity metric. In addition, the image classification method based on the image classification model may include a step S109 of performing a classification task for whether the input image corresponds to each of the multiple labels based on the image classification model trained as described above, and a step S111 of providing a result of the multi-label classification task for the input image according to the image classification task.

Specifically, the computing system 1000 may generate the sample data (S101).

In detail, the computing system 1000 may detect a medical image corresponding to a predetermined criterion in raw-data, and detect a diagnosis report associated with the detected medical image. Here, the raw data may be an electronic medical record (EMR) including multiple medical images and a diagnosis report for the medical images.

For example, the computing system 1000 may 1) determine a specific body organ for the medical image, and 2) when determining an image type (e.g., an RGB image, a radiation image, a computed tomography image, etc.), detect a specific type of image for the specific body organ set through the image detection model as an image of the sample data.

The computing system 1000 may then detect a diagnosis report for the detected medical image of the sample data. For example, the computing system 1000 may determine the associated diagnosis report as the text of the sample data based on metadata or/and a creation template, etc., according to a creation format of the raw data. For example, when the computing system 1000 sets a criterion according to the raw-data creation format in a scheme of extracting a sentence including an ID matching an ID of the medical image and/or a scheme of extracting a sentence described at a front end or a rear end of the medical image, the computing system 1000 may extract the associated diagnosis report as the sample data according to the set criterion. In this case, the computing system 1000 may detect the diagnosis report to include both a paragraph for "findings" and a paragraph for "impression" that contain information in the pathology classification for the medical image.

The computing system 1000 may then match the medical image of the sample data thus extracted with the diagnosis report and store the matched medical image and the diagnosis report as the image-text pair of the sample data.

In addition, the computing system 1000 may perform data augmentation in order to overcome a lack problem of the sample data that is relatively small compared to the training data of the pretrained vision-language model. Here, the data augmentation means that additional sample data is generated and augmented based on the existing sample data.

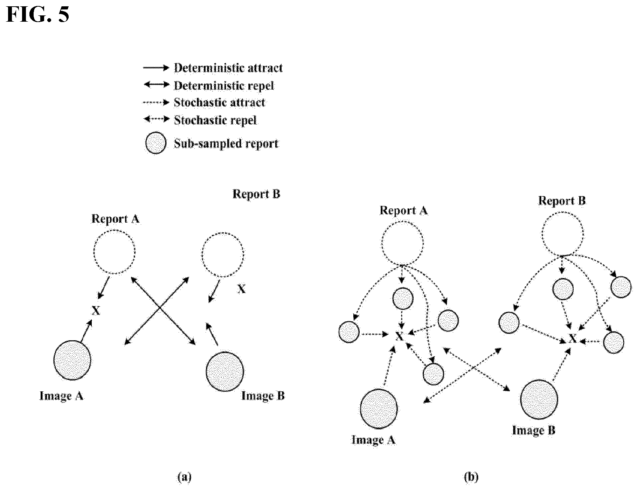

Referring to FIG. 5, when the entire diagnosis report associated with the medical image is set as the text and set as the image-text pair, (a) a matching rate of the similarity between the image and the text becomes too high, such as a diagram, and becomes a negative pair for the diagnosis report for another image that is not associated with the image but has shareable information, and is not suitable for multi-label classification. Relatively, when a sub-report including information for each label is additionally generated for the diagnosis report for the image as in (b) to augment the data, it is possible to perform training so that a similarity may also be matched between the medical image and the sub-report not associated with the medical, and thus texts suitable for learning the medical image of the multi-label nature may be augmented.

According to with the concept, a specific method of the data augmentation according to the embodiment will be described.

The data augmentation according to the embodiment may not be performed for the medical image. In general, schemes such as random cropping, rotation, color conversion, viewpoint change, blurring, random noise addition, etc., which are image augmentation techniques may distort critical meanings in detecting pathologies from medical images.

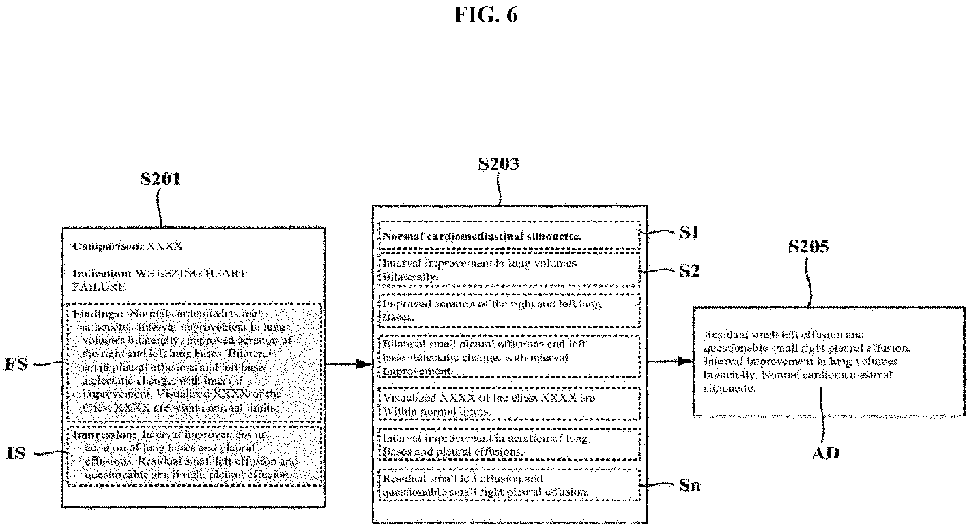

Referring to FIG. 6, the data augmentation according to the embodiment may be random text augmentation performed only for the text (S101).

Specifically, the random text data augmentation method may perform a task of extracting only a paragraph related to the pathology classification from the diagnostic report, thereby removing unnecessary tokens.

For example, the data augmentation method may reconstruct the diagnosis report by extracting a paragraph corresponding to a predetermined table of contents (e.g., "findings" and "impression") (S201). The findings paragraph FS and the impression paragraph IS in the diagnosis report are composed of meaningful contents for the pathology classification, and most of the sentences are composed of short and shaping, so the findings paragraph FS and the impression paragraph IS may be text sample data suitable for the fine tuning.

Then, the data augmentation method may separate each of the sentences S1, S2, ..., and Sn included in the reconstructed diagnosis report into n sentences (S203). Thereafter, in the data augmentation method, a plurality of texts are additionally generated based on n separated sentences S1, S2, ..., and Sn to augment the text data (S205).

In the embodiment, when the number of separated sentences S1, S2, ..., and Sn in the diagnosis report matched with a first medical image is n, and the text is composed of m sentences (n > m), n separated sentences S1, S2, ..., and Sn are randomly sampled and combined to generate nCm sentences an active directory AD for the first medical image. Here, the number of sentences of the active directory AD may be fixed to m, and the number of sub-sentences may be determined differently according to the number of separated sentences of each diagnosis report. In another embodiment, a scheme of randomly determining the number of sentences and randomly sampling the number of sentences of the active directory AD from the separated sentences of the diagnosis report may be taken.

Through the text augmentation method, it is possible to additionally generate multiple active directories ADs that may be positive pairs for the first medical image, and the active directories ADs composed of the multiple positive pairs each express a rich meaning for a specific pathology, so that a rich meaning may be learned for the medical image having the multi-label nature. Further, for other second medical images with overlapped pathologies (labels), negative natures of the active directories ADs may be reduced to train to locate embeddings of active directories ADs with similar pathologies and the second medical image close to each other.

After the text data augmentation is performed, a plurality of active directories ADs are additionally generated for the medical image to enlarge the sample data, so that the sample data may increase in proportion to the additionally generated text data.

Thereafter, the computing system 1000 may map the sample data for the text data augmented for the diagnosis report of the medical image in the positive pair and map the sample data in the negative pair with the other medical image to sufficiently augment positive sample data and negative sample data.

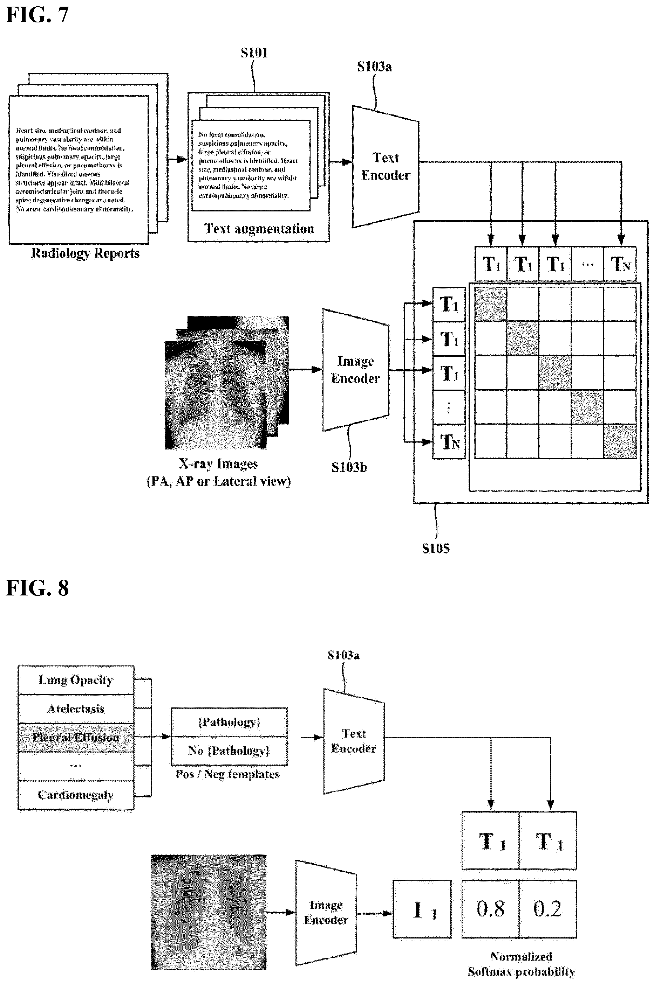

In addition, referring to FIG. 7, the computing system 1000 may control each of the medical images of the sample data to output image embedding vectors (hereinafter, referred to as "embeddings") representing an image feature through an image encoder, and control active directories to output text embedding vectors (hereinafter, referred to as "embeddings") representing a text feature through a text encoder (S103a and S103b).

Next, the computing system 1000 may classify sample data according to a batch size, and match the mapped positive pair and negative pair for the sample data for each classified batch into a positive pair/negative pair of the image embedding and the text embedding to generate a similarity metric (S105).

Here, the similarity may mean a dot product between the image embedding and the text embedding.

That is, the similarity metric may be a matrix that lists embedding samples of the positive pair and embedding samples of the negative pair such that rows and columns of the positive pair match.

Thereafter, the computing system 1000 may perform training according to the loss function to cause a spatial distance between the positive embedding samples to decrease and cause a spatial distance between the negative embedding samples to increase.

That is, contrastive learning may be performed by defining the loss function so as to achieve

$$\underbrace{\mathrm{score}(f(x), f(x^+))}_{\text{Positive samples}} >> \underbrace{\mathrm{score}(f(x), f(x^-))}_{\text{Negative samples}}.$$

For example, the computing system 1000 may perform training by applying InfoNCE Loss which is a first loss function (Equation 1) to the similarity metric.

[Equation 1]

$$l_i^{v \to u} = -\log \frac{\exp\left(\mathbf{sim}(v_i, u_i)/\tau\right)}{\sum_{k=1}^{N} \exp\left(\mathbf{sim}(v_i, u_k)/\tau\right)} \qquad (1)$$

Where v and u mean normalized embedding vectors of the image and the text, respectively, $(v_i, u_i)$ represents the positive embedding sample and $(v_i, u_k)$ represents the negative embedding sample, sim represents a function of calculating the similarity between the embedding vectors, which may be, for example, a cosine similarity, $\tau$ means a learnable temperature, and N represents a batch size.

That is, the image i is paired with one positive text and N-1 negative texts to construct the similarity metric. For $lv \to u$, a degree of loss may be learned through the first loss function such that the text i has a high similarity to the image i.

However, when the first loss function is applied as it is to learn the similarity of the positive pair to be completely high and the similarity of the negative pair to be completely low in the similarity metric, it may be difficult to learn a similarity between positive texts for the image i to be high.

In order to relax this problem, the computing system 1000 may take a scheme of calculating and learning the similarity of the similarity metric according to a second loss function obtained by modifying the function sim for calculating the similarity.

[Equation 2]

$$\mathbf{sim}(v_i, u_j) = \begin{cases} \frac{1}{1+\exp(-\alpha(v_i \cdot u_j - t))} & \text{if } i = j \text{ and} \\ & v_i \cdot u_j \geq t \\ v_i \cdot u_j / 2t & \text{else if } i = j \text{ and} \\ & t > v_i \cdot u_j \geq 0 \\ v_i \cdot u_j & \text{, otherwise} \end{cases}$$

Where $v_i * u_j$ represents a cosine similarity between the image embedding vectors and the text embedding vectors, $\alpha$ represents a slope coefficient of a sigmoid function, and t (0 < t < 1) means the threshold value of the similarity which regards the level of similarity between the sample data as "positive".

That is, when the similarity is greater than t, the similarity may be calculated to quickly approach 1.

In this way, the second loss function, which may perform calculation to relax the matching between the image and the text of the positive pair, may make room for the matching with respect to the image even for texts of the sample that are not positively mapped, thereby training an image classification model in which a plurality of labels are accurately classified into images having the multi-label nature.

In this way, the computing system 1000 may train the image classification model by using two fine tuning techniques described below for the pretrained vision-language model, so that 1) sample data may be sufficiently secured through text data augmentation that is not distorted and has a rich meaning for each pathology, and 2) a loss function in which the matching of positive pairs between sample data is relaxed is applied and leaned to the similarity metric to train the image classification model that accurately classifies whether labels are applied redundantly to medical images having the multi-label nature.

In addition, the computing system 1000 may perform a task of classifying multiple labels for the input image through the image classification model trained as such (S109).

Referring to FIG. 8, the computing system 1000 may perform prompt engineering to perform classification of each multi-label for an input image.

In detail, THE computing system 1000 may define multiple labels for the medical image. For example, each of the pathologies defined in the training data set for the fine tuning may be determined as the label. In the embodiment, the computing system 1000 may input active directories generated by augmenting the text data of the diagnostic report for the medical image into a natural language analysis model to extract detectable pathologies, and determine the extracted pathologies as the labels.

The computing system 1000 may then input each of the labels into a positive/negative template for the input image to generate a sentence for determining whether to classify the labels.

For example, in order to confirm whether a label for a lung consolidation is classified, the computing system 1000 may generate a positive sentence including a lung consolidation positive and a negative sentence including a lung consolidation negative.

Then, the computing system 1000 may input the positive sentence and the negative sentence as the texts into the text encoder of the pretrained image classification model to output the text embedding for the positive sentence and the text embedding of the negative sentence, and input the input image to the image encoder of the pretrained image classification model to output the image embedding.

Then, the computing system 1000 calculates an image embedding-normalized SoftMax similarity (a distance between respective vectors) for each of the text embedding of the positive sentence and the text embedding of the negative sentence, and calculates values of a classification positive similarity and a negative similarity for the corresponding pathology, and classifies the input image into the corresponding pathology when the similarity to the positive is higher, and does not classify the input image into the corresponding pathology when the similarity to the negative is higher.

As such, the computing system 1000 may generate a positive sentence and a negative sentence for each of the multiple labels according to the template, and input the text including the generated positive sentence and negative sentence and the input image into the image classification model to determine whether to classify each label.

Through such prompt engineering, the computing system 1000 may redundantly determine image classifications for multiple labels.

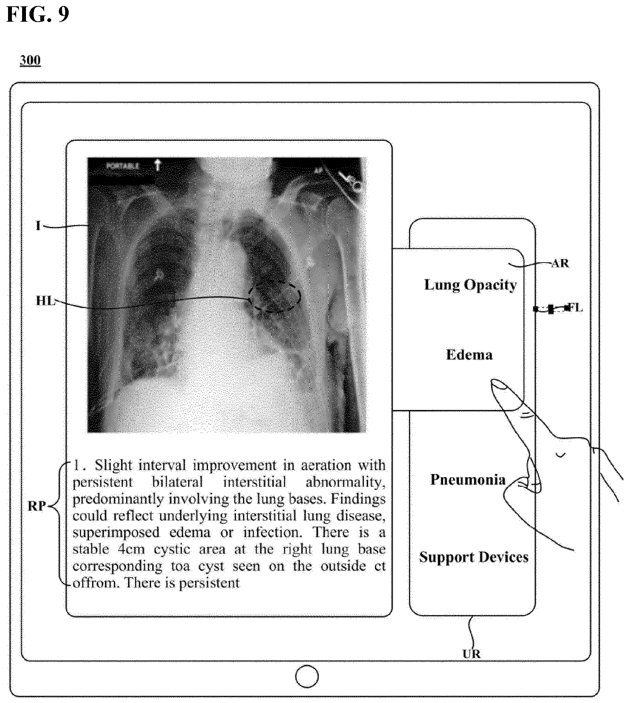

Referring to FIG. 9, the computing system 1000 may display a classified label AR by classifying whether an input image I corresponds to one or more labels (pathologies) as described above, and in this case, provide unclassified pathologies UR to be displayed together to allow the user to confirm a label FL determined by the image classification model. Through this, the user may intuitively grasp a pathology FL which the image classification model may determine for the medical image, and assist in performing efficient diagnosis of the medical image.

In addition, the computing system 1000 may restore and provide a diagnosis report RP based on a text embedding having a high similarity according to whether to apply multiple labels. That is, the computing system 1000 may also generate and provide an artificial intelligence diagnosis report RP for the input image I through an image classification model trained from active directories generated through text augmentation for the diagnosis report.

In addition, the computing system 1000 may calculate an association between at least a partial region of the input image I and the classified label, and highlight (HL) and provide a region of the input image 1 having a high similarity with the classified label, thereby additionally assisting a diagnosis of a user.

Hereinafter, three image classification models are trained by performing fine tuning according to the embodiment of the present invention for three example models among the pretrained vision-language models through the contrastive training, and a zero-shot performance evaluation is performed for each of the three image classification models and the existing preceding image classification models (CheXzero and MedCLIP) so as to prove an effect.

Here, a first image classification model fine-tuned with the example model may be a model fine-tuning a contrastive language-Image pretraining (CLIP)-based framework, a second image classification model may be a model fine-tuning an self-supervision meets language-image pretraining (Slip) based framework, and a third image classification model may be a model fine tuning a unified framework for contrastive language-image pretraining (Uniclip) based framework.

[Table 1]

|  |  | # of Images | # of Reports | # of classes |
|---|---|---|---|---|
| Fine-tuning | MIMIC-CXR [12] | 377,110 | 227,835 | - |
|  | CheXpert$_{valid}$ [11] | 234 | 200 | - |
| Evaluation | CheXpert$_{test}$ [11] | 668 | 500 | 14 |
|  | Open-i [1] | 7,470 | 3,955 | 14 |
|  | PadChest [3] | 39,053 | 26,413 | 61 |
|  | VinDr-CXR [21] | 3,000 | - | 20 |

Table 1 shows a data set used for fine tuning and a data set used for evaluating the zero-shot performance according to the embodiment.

In the embodiment, the training data set used for the fine tuning according to the present invention is MIMIC-CXR.

In addition, a data set for verification of CheXpert, a data set of PadChest, an Open-i chest X-ray data set, and VinDr-CXR are used as test data sets.

Results of performing the zero-shot performance evaluation by utilizing the test data sets for the first image classification model, the second image classification model, a third image classification model, and a first preceding image classification model and a second preceding image classification model are shown in Table 2 below.

[Table 2]

| | CheXpert | | Open-i | | PadChest | VinDr-CXR |
| | avg. 5 AUC | avg. total AUC | avg. 5 AUC | avg. total AUC | avg. total AUC | avg. total AUC |
|---|---|---|---|---|---|---|
| CLIP | $0.870 \pm 0.006$ | $0.771 \pm 0.001$ | $0.785 \pm 0.030$ | $0.691 \pm 0.010$ | $0.685 \pm 0.026$ | $0.801 \pm 0.020$ |
| CLIP w/ ours | $\mathbf{0.885 \pm 0.007}$ | $\mathbf{0.789 \pm 0.017}$ | $\mathbf{0.808 \pm 0.008}$ | $\mathbf{0.708 \pm 0.013}$ | $\mathbf{0.725 \pm 0.014}$ | $\mathbf{0.842 \pm 0.007}$ |
| SLIP | $0.831 \pm 0.008$ | $0.722 \pm 0.008$ | $0.760 \pm 0.008$ | $0.666 \pm 0.004$ | $0.684 \pm 0.004$ | $0.799 \pm 0.018$ |
| SLIP w/ ours | $\mathbf{0.884 \pm 0.009}$ | $\mathbf{0.779 \pm 0.020}$ | $\mathbf{0.810 \pm 0.016}$ | $\mathbf{0.719 \pm 0.018}$ | $\mathbf{0.741 \pm 0.005}$ | $\mathbf{0.831 \pm 0.009}$ |
| UniCLIP | $0.851 \pm 0.008$ | $0.741 \pm 0.037$ | $0.744 \pm 0.032$ | $0.643 \pm 0.027$ | $0.684 \pm 0.009$ | $0.759 \pm 0.019$ |
| UniCLIP w/ ours | $\mathbf{0.888 \pm 0.006}$ | $\mathbf{0.802 \pm 0.018}$ | $\mathbf{0.788 \pm 0.016}$ | $\mathbf{0.694 \pm 0.006}$ | $\mathbf{0.717 \pm 0.011}$ | $\mathbf{0.843 \pm 0.016}$ |
| CheXzero | $0.844 \pm 0.008$ | $0.733 \pm 0.008$ | $0.771 \pm 0.006$ | $0.684 \pm 0.006$ | $0.702 \pm 0.011$ | $0.771 \pm 0.024$ |
| MedCLIP* | $0.880$ | $0.829$ | $0.794$ | $0.728$ | $0.728$ | $0.829$ |

*: use label information defined in CheXpert for training

Table 2 shows that the fine tuning technique according to the embodiment of the present invention continuously improves the zero-shot pathology classification performance through an average 5.77% increase in area under ROC curve (AUROC) according to whether the first to third image classification models are subjected to fine tuning, thereby demonstrating a superiority of the fine tuning technique.

[Table 3]

| | Text aug | Relax loss | Atelectasis | Cardiomegaly | Consolidation | Edema | Pleural Effusion | avg. 5 AUC | avg. total AUC |
|---|---|---|---|---|---|---|---|---|---|
| CLIP | | | $0.828 \pm 0.016$ | $0.845 \pm 0.012$ | $0.895 \pm 0.007$ | $0.861 \pm 0.016$ | $0.878 \pm 0.020$ | $0.861 \pm 0.002$ | $0.787 \pm 0.044$ |
| | | ✓ | $0.858 \pm 0.009$ | $0.815 \pm 0.013$ | $0.889 \pm 0.023$ | $0.875 \pm 0.022$ | $\mathbf{0.890 \pm 0.006}$ | $0.865 \pm 0.002$ | $0.794 \pm 0.045$ |
| | ✓ | | $\mathbf{0.864 \pm 0.004}$ | $\mathbf{0.867 \pm 0.008}$ | $0.904 \pm 0.015$ | $\mathbf{0.903 \pm 0.003}$ | $0.877 \pm 0.021$ | $\mathbf{0.883 \pm 0.005}$ | $\underline{0.820 \pm 0.001}$ |
| | ✓ | ✓ | $0.859 \pm 0.006$ | $0.840 \pm 0.016$ | $\mathbf{0.913 \pm 0.010}$ | $0.881 \pm 0.009$ | $0.869 \pm 0.012$ | $\underline{0.872 \pm 0.005}$ | $\mathbf{0.824 \pm 0.003}$ |
| SLIP | | | $0.760 \pm 0.046$ | $0.788 \pm 0.008$ | $0.877 \pm 0.028$ | $0.847 \pm 0.021$ | $\mathbf{0.902 \pm 0.010}$ | $0.835 \pm 0.010$ | $0.791 \pm 0.015$ |
| | | ✓ | $0.826 \pm 0.010$ | $0.828 \pm 0.010$ | $0.899 \pm 0.006$ | $0.873 \pm 0.013$ | $0.899 \pm 0.010$ | $\underline{0.865 \pm 0.005}$ | $\mathbf{0.828 \pm 0.003}$ |
| | ✓ | | $0.830 \pm 0.022$ | $0.837 \pm 0.021$ | $0.864 \pm 0.042$ | $0.899 \pm 0.008$ | $0.876 \pm 0.013$ | $0.861 \pm 0.004$ | $0.800 \pm 0.015$ |
| | ✓ | ✓ | $\mathbf{0.845 \pm 0.014}$ | $\mathbf{0.855 \pm 0.018}$ | $\mathbf{0.904 \pm 0.014}$ | $\mathbf{0.905 \pm 0.016}$ | $0.868 \pm 0.009$ | $\mathbf{0.875 \pm 0.006}$ | $\underline{0.819 \pm 0.028}$ |
| UniCLIP | | | $0.774 \pm 0.060$ | $0.833 \pm 0.012$ | $0.869 \pm 0.016$ | $0.856 \pm 0.033$ | $0.882 \pm 0.020$ | $0.843 \pm 0.010$ | $0.750 \pm 0.016$ |
| | | ✓ | $0.840 \pm 0.009$ | $0.818 \pm 0.021$ | $0.870 \pm 0.012$ | $0.869 \pm 0.014$ | $\mathbf{0.897 \pm 0.021}$ | $0.859 \pm 0.008$ | $\underline{0.808 \pm 0.023}$ |
| | ✓ | | $0.860 \pm 0.012$ | $\mathbf{0.862 \pm 0.010}$ | $0.900 \pm 0.019$ | $0.879 \pm 0.005$ | $0.887 \pm 0.012$ | $\underline{0.877 \pm 0.009}$ | $0.790 \pm 0.020$ |
| | ✓ | ✓ | $\mathbf{0.870 \pm 0.007}$ | $0.844 \pm 0.007$ | $\mathbf{0.911 \pm 0.002}$ | $\mathbf{0.886 \pm 0.014}$ | $0.882 \pm 0.017$ | $\mathbf{0.878 \pm 0.001}$ | $\mathbf{0.825 \pm 0.028}$ |

*: use label information defined in CheXpert for training

In addition, Table 3 shows experiments on classification accuracy by various pathologies (diseases) of the proposed method in CheXpert's test set (average $\pm$ standard). For each framework, a highest performance is indicated in bold letters and a second highest performance is indicated to be underlined. Referring to Table 3, it may also be clearly understood that the zero-shot performance is improved when the text data augmentation and the relaxation loss function are applied in the fine tuning technique.

In particular for the CheXpert data set, fine-tuning the CLIP with the image classification model training method according to the present invention sometimes shows a slightly better performance than the committee certified radiologist in detecting the five major diseases with an F1 score of 0.619 vs. 0.625 and an MCC of 0.530 vs. 0.544, which are not tabulated.

As such, the present invention may provide an image classification model including a neural network that improves downstream zero-shot performance by fine-tuning an image classification model pretrained with the general domain image-text pair without a separate labeling task.

As a result of performing zero-shot pathology classification after fine tuning four chest X-ray data sets and three pretrained models (average 5.77% AUROC increase), the image classification model of the present invention may continuously show dramatically improved zero-shot pathological classification performance. In addition, the image classification model of the present invention may also derive an experimental result showing a performance much similar to or better than that of the radiologist certified by the committee.

The embodiments described above are implemented in a form of a program instruction which may be executed through various computer components and may be recorded in the computer readable recording media. The computer readable recording media may include singly a program instruction, a data file, or a data structure or a combination thereof. The program instruction recorded in the computer readable recording medium may be specially designed and configured for the present invention, or may be publicly known to and used by those skilled in the computer software field. Examples of the computer readable recording media may include a hardware device particularly configured to store and execute program instructions, magnetic media such as hard disks, floppy disks, and magnetic tape, optical recording media such as CD-ROM disks and DVD, magneto-optical media such as floptical disks, ROM, RAM, and flash memories. Examples of the program instructions include a high-level language code executable by a computer by using an interpreter, and the like, as well as a machine language code created by a compiler. The hardware devices may be changed to one or more software modules in order to perform the processing according to the present invention, and an opposite situation thereof is available.

Specific executions described in the present invention are embodiments and the scope of the present invention is not limited even by any method. For brevity of the specification, descriptions of conventional electronic configurations, control systems, software, and other functional aspects of the systems may be omitted. Further, connection or connection members of lines among components exemplarily represent functions connections and/or physical or circuitry connections and may be represented as various functional connections, physical connections, or circuitry connections which are replaceable or added in an actual device. Further, unless otherwise specified, such as "essential", "important", etc., the connections may not be components particularly required for application of the present invention.

[Industrial Applicability]

The present invention is a method for training an image classification model including a neural network through a computing device, and classifying images having the multi-label nature by label using the trained image classification model, and thus has an industrial applicability.

**Claims**

1.   A method for training image classification model by a computing system including a memory and a processor, the method comprising:

augmenting text data in sample data of a plurality of image-text pairs;
training the image classification model by fine-turning a pretrained vision-language model based on the data-augmented texts and an image; and
providing the trained image classification model.

2.   The method of claim 1, wherein the augmenting of the text data in the sample data includes

obtaining raw data composed of a plurality of multi-label images, and reports including descriptions of the multi-label images;
detecting a multi-label image according to a preset criterion in the raw data, and
detecting a report associated with the detected multi-label image.

3.   The method of claim 2, wherein the augmenting of the text data in the sample data includes
extracting one or more paragraphs corresponding to the description of classifying the multiple labels from the detected report to generate a reconstructed report.

4.   The method of claim 1, wherein the augmenting of the text data in the sample data includes

obtaining a plurality of multi-label images and a plurality of reports associated with respective images,
separating sentences included in the plurality of reports to detect n sentences, and
generating a plurality of active directories based on the n detected sentences.

5.   The method of claim 4, wherein the generating of the plurality of active directories includes
generating the plurality of active directories by a scheme of randomly sampling and combining the n detected sentences.

**6.** The method of claim 4, wherein the training of the image classification model by fine-tuning the pretrained vision language model includes

mapping each of a plurality of active directories generated from a first report for a first-label image to the first multi-label image in a positive pair, and mapping each active directory to another multi-label image in a negative pair to generate augmented sample data.

**7.** The method of claim 6, wherein the training of the image classification model by fine-tuning the pretrained vision language model includes

classifying the augmented sample data based on a batch size,
outputting an image embedding by inputting multi-label images of sample data for each batch into an image encoder,
outputting a text embedding by inputting active directories of the sample data for each batch into a text encoder, and
generating a similarity metric by listing the image embedding and the text embedding based on the mapped positive pair and negative pair.

**8.** The method of claim 7, wherein the training of the image classification model by fine-tuning the pretrained vision language model includes

calculating a similarity in the generated similarity metric through a relaxing loss function, and training the image classification model so that a similarity between the positive pairs is relaxed.

**9.** The method of claim 8, wherein the relaxed loss function is

$$
\mathbf{sim}(v_i, u_j) = \begin{cases} \dfrac{1}{1+\exp(-\alpha(v_i \cdot u_j - t))} & \text{if } i = j \text{ and} \\[4pt] & v_i \cdot u_j \geq t \\[8pt] v_i \cdot u_j / 2t & \text{else if } i = j \text{ and} \\[4pt] & t > v_i \cdot u_j \geq 0 \\[8pt] v_i \cdot u_j & \text{, otherwise} \end{cases},
$$

where v and u mean normalized embedding vectors of the image and the text, respectively, (vi, $u_j$) represents the positive embedding sample and ($v_i$, $u_k$) represents the negative embedding sample, sim represents a function of calculating the similarity between the embedding vectors, which may be, for example, a cosine similarity, $\tau$ means a learnable temperature, and N represents a batch size.

**10.** A method for classifying an input image by using an image classification model trained according to the image classification model training method of claim 1.

**11.** The method of claim 10, comprising:

obtaining an input image from a user computing device;
inputting the obtained input image, and a plurality of sentences generated for multiple labels through prompt engineering into the image classification model;
detecting whether to apply respective labels to the plurality of sentences from the image classification model; and
providing a result according to detecting whether to apply the respective labels.

**12.** The method of claim 11, wherein the multi-label image is a medical image, and
the image classification model is a model that determines whether to apply a plurality of pathologies tot eh medical image.

**13.** A computing system for individually classifying a plurality of pathologies for an input medical image having a multi-label nature, the computing device comprising:

a memory storing an image classification model classifying a plurality of pathologies for the input medical image;

and
one or more processors operatively combining the image classification mode and the data of the memory, wherein the one or more processors
obtain raw data composed of a plurality of multi-label images, and reports including descriptions of the multi-label images,
detect a multi-label image according to a preset criterion in the obtained raw data,
detect a report associated with the detected multi-label image,
individually separating sentences included in the plurality of reports to detect n sentences,
augment text data by generating a plurality of active directories based on the n detected sentences,
train an image classification model by fine-turning a vision-language model pretrained based on the data-augmented texts and an image, and
provide the trained image classification model.

14. The system for training an image classification model for a multi-label image of claim 13, wherein the at least one processor

generates the plurality of active directories by a scheme of randomly sampling and combining the n detected sentences, and
maps each of a plurality of active directories generated from a first report for a first-label image to the first multi-label image in a positive pair, and maps each active directory to another multi-label image in a negative pair to generate augmented sample data.

15. The system for training an image classification model for a multi-label image of claim 14, wherein

the at least one processor
outputs an image embedding by inputting multi-label images of sample data for each batch in the generated sample data into an image encoder,
output a text embedding by inputting active directories of the sample data for each batch into a text encoder,
generate a similarity metric by listing the image embedding and the text embedding based on the mapped positive pair and negative pair, and
calculate a similarity in the generated similarity metric through a relaxing loss function, and training the image classification model so that a similarity between the positive pairs is relaxed.

# FIG. 1

<u>1000</u>

**110** User computing device

**112** Memory
**113** Data
**111** Processor(s)
**114** Instructions

**120** Machine learning model(s)

**121** User input component

**170** Network

**130** Server computing system
**132** Memory
**133** Data
**131** Processor(s)
**134** Instructions
**140** Machine learning model(s)

**150** Training computing system
**152** Memory
**153** Data
**151** Processor(s)
**154** Instructions
**160** Model trainer
**161** Training data

**FIG. 2**

## FIG. 3

**200**

Computing device

Central Intelligence Layer

| Application 1 | ⟷ | Model 1 | | Sensor(s) |

| Application 2 | ⟷ | Model 2 | | Context manager |

| Application 3 | ⟷ | Model 3 | | Device state |

⋮ ⋮

| Application N | ⟷ | Model N | | Additional component(s) |

Central device data layer

**FIG. 4**

AUGMENT IMAGE-TEXT DATA, AND GENERATE
POSITIVE PAIRS AND NEGATIVE PARIS — S101

EXTRACT FEATURE BY ENCODING IMAGE-TEXT — S103

GENERATE SIMILARITY METRIC OF IMAGE-TEXT
FEATURE ACCORDING TO POSITIVE/NEGATIVE
PAIRS — S105

PERFORM TRAINING BY APPLYING RELAXED
LOSS BETWEEN POSITIVE PAIRS TO GENERATED
SIMILARITY METRIC — S107

PERFORM MULTI-LABEL CLASSIFICATION TASK OF
INPUT IMAGE BASED O N IMAGE CLASSIFICATION
MODEL INCLUDING TRAINED NEURAL NETWORK — S109

PROVIDE MULTI-LABEL CLASSIFICATION TASK
RESULT CLASSIFIED BY IMAGE CLASSIFICATION
MODEL — S111

# FIG. 5

(a)

(b)

FIG. 6

S201

S203

S205

**Comparison:** XXXX

**Indication:** WHEEZING/HEART FAILURE

**Findings:** Normal cardiomediastinal silhouette. Interval improvement in lung volumes bilaterally. Improved aeration of the right and left lung bases. Bilateral small pleural effusions and left base atelectatic change, with interval improvement. Visualized XXXX of the Chest XXXX are within normal limits.

**Impression:** Interval improvement in aeration of lung bases and pleural effusions. Residual small left effusion and questionable small right pleural effusion.

FS

IS

**Normal cardiomediastinal silhouette.** — S1

Interval improvement in lung volumes Bilaterally. — S2

Improved aeration of the right and left lung Bases.

Bilateral small pleural effusions and left base atelectatic change, with interval Improvement.

Visualized XXXX of the chest XXXX are Within normal limits.

Interval improvement in aeration of lung Bases and pleural effusions.

Residual small left effusion and questionable small right pleural effusion. — Sn

Residual small left effusion and questionable small right pleural effusion. Interval improvement in lung volumes bilaterally. Normal cardiomediastinal silhouette.

AD

EP 4 618 046 A1

19

## FIG. 7

Radiology Reports

Text augmentation

Text Encoder

X-ray Images
(PA, AP or Lateral view)

Image Encoder

## FIG. 8

Lung Opacity

Atelectasis

Pleural Effusion

...

Cardiomegaly

{Pathology}

No {Pathology}

Pos / Neg templates

Text Encoder

Image Encoder

Normalized
Softmax probability

**FIG. 9**

300

1. Slight interval improvement in aeration with persistent bilateral interstitial abnormality, predominantly involving the lung bases. Findings could reflect underlying interstitial lung disease, superimposed edema or infection. There is a stable 4cm cystic area at the right lung base corresponding toa cyst seen on the outside ct offrom. There is persistent

Lung Opacity

Edema

Pneumonia

Support Devices

## INTERNATIONAL SEARCH REPORT

| International application No. |
| --- |
| **PCT/KR2023/018499** |

### A. CLASSIFICATION OF SUBJECT MATTER

**G06V 20/69**(2022.01)i; **G06V 10/80**(2022.01)i; **G06V 10/774**(2022.01)i; **G06V 30/19**(2022.01)i; **G06V 10/46**(2022.01)i; **G06V 10/82**(2022.01)i

According to International Patent Classification (IPC) or to both national classification and IPC

### B. FIELDS SEARCHED

Minimum documentation searched (classification system followed by classification symbols)

G06V 20/69(2022.01); A61B 5/00(2006.01); G06F 16/31(2019.01); G06F 16/55(2019.01); G06F 16/958(2019.01); G06N 20/00(2019.01); G06N 3/04(2006.01); G06N 3/08(2006.01); G06V 10/46(2022.01); G06V 10/75(2022.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 병리(pathology), 이미지 분류(image classification), 텍스트(text), 파인 튜닝(fine tuning), 텍스트 데이터 확대(text data augmentation)

### C. DOCUMENTS CONSIDERED TO BE RELEVANT

| Category* | Citation of document, with indication, where appropriate, of the relevant passages | Relevant to claim No. |
| --- | --- | --- |
| A | KR 10-2022-0147550 A (BEIJING BAIDU NETCOM SCIENCE TECHNOLOGY CO., LTD.) 03 November 2022 (2022-11-03) <br> See paragraphs [0017], [0024]-[0025] and [0042]; claim 1; and figures 2-3. | 1-15 |
| A | KR 10-2022-0011294 A (BUZZ&BEYOND CO., LTD.) 28 January 2022 (2022-01-28) <br> See paragraphs [0016], [0058] and [0061]; and figure 2. | 1-15 |
| A | KR 10-2022-0131808 A (DAEGU GYEONGBUK INSTITUTE OF SCIENCE AND TECHNOLOGY) 29 September 2022 (2022-09-29) <br> See paragraphs [0049]-[0050] and [0053]; and figures 1-2. | 1-15 |
| A | KR 10-2020-0095254 A (NSDEVIL) 10 August 2020 (2020-08-10) <br> See paragraphs [0022]-[0033]; and figure 2. | 1-15 |
| A | KR 10-2020-0135171 A (LUNIT INC.) 02 December 2020 (2020-12-02) <br> See paragraphs [0059]-[0075]; and figures 2-3. | 1-15 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **22 February 2024** | **22 February 2024** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

| International application No. |
| --- |
| **PCT/KR2023/018499** |

| Patent document cited in search report | | | Publication date (day/month/year) | Patent family member(s) | | | Publication date (day/month/year) |
| --- | --- | --- | --- | --- | --- | --- | --- |
| KR | 10-2022-0147550 | A | 03 November 2022 | CN | 114549874 | A | 27 May 2022 |
| | | | | JP | 2022-191412 | A | 27 December 2022 |
| | | | | JP | 7403605 | B2 | 22 December 2023 |
| | | | | US | 2023-0196716 | A1 | 22 June 2023 |
| KR | 10-2022-0011294 | A | 28 January 2022 | None | | | |
| KR | 10-2022-0131808 | A | 29 September 2022 | KR | 10-2611121 | B1 | 07 December 2023 |
| KR | 10-2020-0095254 | A | 10 August 2020 | KR | 10-2199480 | B1 | 06 January 2021 |
| KR | 10-2020-0135171 | A | 02 December 2020 | KR | 10-2245219 | B1 | 27 April 2021 |
| | | | | US | 11334994 | B2 | 17 May 2022 |
| | | | | US | 11663718 | B2 | 30 May 2023 |
| | | | | US | 2020-0372641 | A1 | 26 November 2020 |
| | | | | US | 2022-0237793 | A1 | 28 July 2022 |
| | | | | US | 2023-0252626 | A1 | 10 August 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ALEC RADFORD ; JONG WOOK KIM ; CHRIS HALLACY ; ADITYA RAMESH ; GABRIEL GOH ; SANDHINI AGARWAL ; GIRISH SASTRY ; AMANDA ASKELL ; PAMELA MISHKIN ; JACK CLARK**. *Learning Transferable Visual Models From Natural Language Supervision*, 26 February 2021
- **EKIN TIU ; ELLIE TALIUS ; PUJAN PATEL ; CURTIS P LANGLOTZ ; ANDREW Y NG ; PRANAV RAJPURKAR.** Expert-level detection of pathologies from unannotated chest x-ray images via selfsupervised learning.. *Nature Biomedical Engineering*, 2022, 1-8
- **ZIFENG WANG ; ZHENBANG WU ; DINESH AGARWAL ; JIMENG SUN.** Medclip: Contrastive learning from unpaired medical images and text.. *EMNLP*, 2022
- **YUHAO ZHANG ; HANG JIANG ; YASUHIDE MIURA ; CHRISTOPHER D MANNING ; CURTIS P LANGLOTZ.** Contrastive learning of medical visual representations from paired images and text.. *arXiv preprint arXiv:2010.00747*, 2020

- **YEN NHI TRUONG VU ; RICHARD WANG ; NIRANJAN BALACHANDAR ; CAN LIU ; ANDREW Y NG ; PRANAV RAJPURKAR.** Medaug: Contrastive learning leveraging patient metadata improves representations for chest x-ray interpretation. *Machine Learning for Healthcare Conference*, 755-769
- *PMLR*, 2021
- **SHEKOOFEH AZIZI ; BASIL MUSTAFA ; FIONA RYAN ; ZACHARY BEAVER ; JAN FREYBERG ; JONATHAN DEATON ; AARON LOH ; ALAN KARTHIKESALINGAM ; SIMON KORNBLITH ; TING CHEN et al.** Big self-supervised models advance medical image classification.. *Proceedings of the IEEE/CVF International Conference on Computer Vision*, 2021, 3478-3488
- **MARIA DE LA IGLESIA-VAY'A**. Padchest: A large chest x-ray image dataset with multi-label annotated reports.. *Medical image analysis*, 2020, vol. 66, 101797